# EUROPEAN PATENT SPECIFICATION

(11) **EP 1 198 586 B1**
(45) Date of publication and mention of the grant of the patent: **03.03.2010**
(21) Application number: 00946853.9
(22) Date of filing: 26.06.2000
(51) Int. Cl.: C12Q 1/00, C12Q 1/68, C12P 1/00, G01N 33/53

(54) **AN IN VIVO LIBRARY-VERSUS-LIBRARY SELECTION OF OPTIMIZED PROTEIN-PROTEIN INTERACTIONS**
EINE IN VIVO "BIBLIOTHEK-GEGEN-BIBLIOTHEK-SELEKTION" VON OPTIMIERTEN PROTEIN-PROTEIN-WECHSELWIRKUNGEN
SELECTION IN VIVO BIBLIOTHEQUE CONTRE BIBLIOTHEQUE D'INTERACTIONS PROTEINE-PROTEINE OPTIMISEES

(30) Priority: 26.06.1999 US 141210 P; 26.06.2000 US 603885
(43) Date of publication of application: 24.04.2002
(73) Proprietor: Odyssey Thera, Inc., San Ramon, CA 94583 (US)
(72) Inventor: MICHNICK, Stephen W., Westmount Québec H3C 2M2 (CA); PELLETIER, Joelle, Nina, Westmount, Québec H3Y 3KA (CA); PLUCKTHUN, Andreas, Biochemisches Institut, CH-8057 Zurich (CH); ARNDT, Katja M., 79108 Freiburg (DE)
(74) Representative: Schüssler, Andrea
(86) International application number: PCT/US2000/017436
(87) International publication number: WO 2001/000866

(56) References cited:
- WO-A-00/17221
- WO-A-01/00814
- WO-A-92/20791
- WO-A-98/34120
- US-A- 5 610 015
- US-A- 5 750 667
- US-A- 5 925 523
- US-A- 5 994 519
- US-A- 6 004 746
- ARNDT K M ET AL: "In-vivo selection of interacting peptide libraries by selectively-infective phages" FASEB JOURNAL, FED. OF AMERICAN SOC. FOR EXPERIMENTAL BIOLOGY, BETHESDA, MD, US, vol. 11, no. 9, 1997, page A1327 XP002156485 ISSN: 0892-6638
- HODGES R S: "DE NOVO DESIGN OF ALPHA-HELICAL PROTEINS: BASIC RESEARCH TO MEDICALAPPLICATIONS" BIOCHEMISTRY AND CELL BIOLOGY. BIOCHIMIE ET BIOLOGIE CELLULAIRE, XX, XX, vol. 74, no. 2, 1996, pages 133-154, XP000605834 ISSN: 0829-8211
- ARNDT KATJA M ET AL: "A heterodimeric coiled-coil peptide pair selected in vivo from a designed library-versus-library ensemble." JOURNAL OF MOLECULAR BIOLOGY, vol. 295, no. 3, 21 January 2000 (2000-01-21), pages 627-639, XP002156489 ISSN: 0022-2836
- PELLETIER ET AL: "An in vivo library-versus-library selection of optimized protein-protein interactions" NATURE BIOTECHNOLOGY, NATURE PUBLISHING, US, vol. 17, July 1999 (1999-07), pages 6836-90, XP002156488 ISSN: 1087-0156
- JUNG S ET AL: "Selectively infective phage (SIP) technology: scope and limitations" JOURNAL OF IMMUNOLOGICAL METHODS, ELSEVIER SCIENCE PUBLISHERS B.V.,AMSTERDAM, NL, vol. 231, no. 1-2, 10 December 1999 (1999-12-10), pages 93-104, XP004187637 ISSN: 0022-1759

## Description

The following abbreviations are used through the present specification: mDHFR, murine dihydrofolate reductase; WinZip: dominant zipper pairs obtained from competition selection: WinZip-A1B1: original pair selected, comprising peptide A1 from library A and peptide B1 from libraryB; WinZip-A1B2 and WinZip-A2B1: optimized pairs comprising the original partner A1 or B1 and the new partner B2 or A2, respectively.

### FIELD OF THE INVENTION

Many of the problems currently being studied in molecular biology and biochemistry share a common factor they are governed by essential molecular interactions, which are often protein-protein interactions. Important examples are the identification and functional characterization of novel gene products, the dissection of proteins into structural or functional motifs and the testing of hypotheses about the physical basis of protein-protein complementarity, whether in naturally-occuring proteins or in designed products. Our ability to address these problems has been transformed by the development of peptide- and protein-library screening techniques such as the yeast two-hybrid strategy^{1,2} and phage display,³ where a library of proteins is panned against a "bait" protein. However, as the study of interacting partners is a "two-dimensional" problem influenced by variations in either partner, it would be advantageous to pan a library of proteins not against a single bait protein, but against a second library of proteins. To date, no large-scale library-*vs*-library selection of protein-protein interactions has been reported, because the available strategies are not amenable to this in any practical way.

WO 98/34120 A1 concerns a protein fragment complementation assay procedure for screening cDNA/protein expression libraries for interactions with a given target. In this context the use of a prokaryotic survival assay is disclosed.

WO 92/20791 A1 relates to the formation of H and L chains of antibodies, and the expression of libraries of each of these components to allow individual components of each type to interact and form novel interacting pairs.

Arndt et al., FASEB J., 11(9), p. AI 327 (1997) is a short abstract about the in-vivo selection of interacting pepetide libraries by selectively-infective phages.

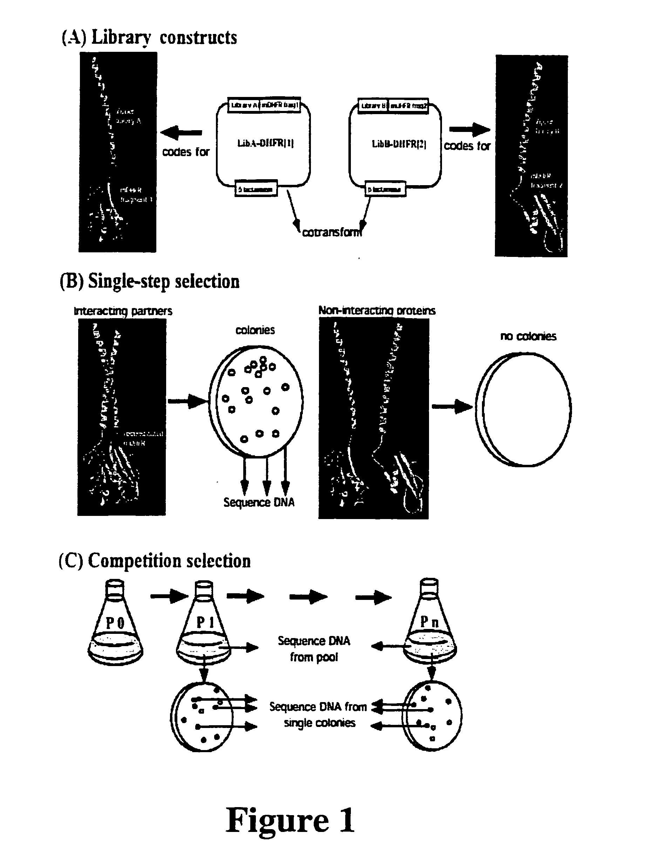

The present invention describes a strategy for library-*vs*-library screening in intact cells based on the folding of murine enzyme dihydrofolate reductase (mDHFR) from complementary fragments⁴⁻¹. DHFR was genetically dissected into two rationally designed fragments, each of which can be fused to a library of proteins or peptides (Fig. 1A). Members of one library which heterodimerize with a member of the other library drive the reassembly of the mDHFR fragments, resulting in reconstitution of enzymatic activity (Fig. 1B). Activity is detected *in vivo* using an *E. coli*-based selection assay, where the bacterial DHFR is specifically inhibited with trimethoprim, preventing biosynthesis of purines, thymidylate, methionine and pantothenate, and therefore cell division. The reconstituted mDHFR, which is insensitive to the low trimethoprim concentration present in selection, restores the biosynthetic reactions required for bacterial propagation. As a result, the interaction between library partners is directly linked to cell survival and detected by colony formation. We have previously demonstrated the utility of this strategy with GCN4 leucine zipper-forming peptides, as well as with larger heterodimerizing partner proteins⁵ with K_{D}s ranging between 3 and 160 nM ^{8,9} although the affinity limits have not been determined.

In this study we demonstrate a large-scale library-*vs*-library selection based on the mDHFR fragment complementation assay: we screened two designed libraries of complementary heterodimeric coiled-coil forming sequences against each other. Our goal was to determine if the strategy would select interacting peptide pairs in which amino acids at the semi-randomized positions are similar to those observed in naturally-occuring or successfully designed coiled-coils which form stable heterodimers (see ¹⁰⁻¹², for example). Further, it is not currently possible to predict sequences of coiled coil-forming peptides that will simultaneously have high stability and heterospecificity as well as advantageous in-vivo properties, such as resistance to proteases. In the present approach, the heterodimerizing peptides will have such characteristics by the nature of their selection. This is crucial to practical applications of optimal interacting heterodimers for *in vivo* studies of protein oligomerization, *e.g*. the design of bispecific miniantibodies¹³.

Three selection strategies were tested here. each having a different level of stringency. In the lowest stringency selection. we screened two expressed libraries against each other in a *single-step selection* (Fig. 1B), thereby identifying all interacting polypeptide partners. In the second strategy, we increased the selection stringency by using a mutant DHFR fragment (Ile114Ala) which prevents stable reassembly of DHFR from its fragments⁵ and should thus require more efficiently heterodimerizing, as opposed to homodimerizing, interacting partners to drive enzyme reconstitution. Finally, we introduced competitive metabolic selection, where clones obtained with the second strategy were pooled and passaged through several rounds of *competition selection,* in order to enrich for the optimally heterodimerizing partners.

By simultaneously screening two libraries against each other, we illustrate the advantages of screening a large, combinatorial sequence space in identifying stably heterodimerizing pairs. We partially sampled a sequence space of 1.72 x 10¹⁰ combinations to select novel leucine zipper pairs with characteristics consistent with stable and specific heterodimerization. We directly demonstrate that the bias toward stability and specificity increased with increasing stringency of selection and observe the rate at which different sequence positions reach a consensus. Additionally, the *in-vivo* selection process ensures that solubility and stability toward proteolysis are essential for selection, yielding products ideally suited to *in-vivo* applications.

### DESCRIPTION OF THE FIGURES

**Figure 1****:** (A) DNA constructs code for fusions between library proteins (shown as α-helical leucine zippers) and either fragment of murine DHFR (mDHFR). Fusions were created using either the wild-type or the mutant mDHFR fragment 2 (Ile114Ala), yielding LibA-DHFR[1] and LibB-DHFR[2] or LibB-DHFR[2:I114A], respectively. (B) Principle of the mDHFR-fragment complementation assay: *E. coli* cells are cotransformed with both fusion libraries in minimal medium, in the presence of IPTG (for induction of expression) and trimethoprim (for inhibition of the bacterial DHFR). If the library proteins heterodimerize, mDHFR can fold from the individual fragments resulting in active enzyme and bacterial growth. Both mDHFR fragments must be present, and dimerization of the fused proteins is essential, in order for cell propagation to be possible. No growth is observed if any of these conditions is not fulfilled⁵. The surviving colonies are the result of "single-step selection" and can be directly analyzed by DNA sequencing. (C) "Competition selection" is undertaken by pooling colonies from (B) in selective, liquid culture (passage 0 or PO), propagating the cells and diluting into fresh selective medium for further passages. An aliquot can be plated and the resulting colonies analyzed by DNA sequencing.

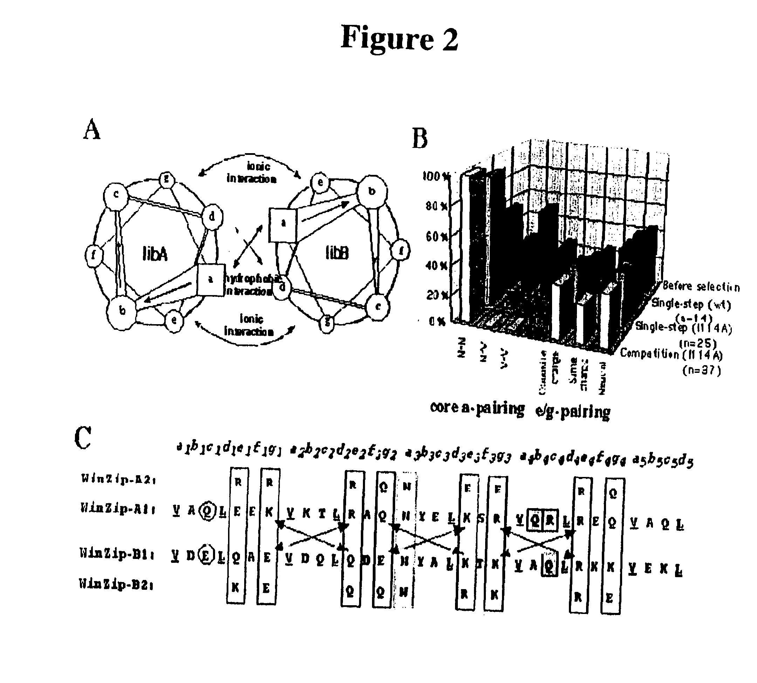

**Figure 2****:** (A) Schematic representation of a leucine zipper pair visualized from the N-terminus illustrating e/g-interactions and the hydrophobic core formed by the a- and d-positions. (B) Distribution of residues at the semi-randomized positions throughout selection. The number of zipper pairs sequenced is given in parentheses, save "Before selection" where the theoretical distribution is reported. Each pair carries one core a-pair and 6 e/g-pairs. Neutral e/g-pairs have one or both residues as Gln. In "Competition (I114A)" only clones from P6 to P12 (not from earlier passages) were considered for analysis. Thus, 37 individual clones were identified, giving rise to 10 unique sequences due to multiple occurence of the enriched clones. The distributions were calculated according to the frequency of sequence occurence (n=37). (C) Leucine zipper sequences obtained after competition selection and chain shuffling. The heptad positions (a to g) are followed by the heptad number (1 to 5). Invariant residues from GCN4 are underlined. Clear boxes indicate the semi-randomized e- and g-positions (black outline) and core α-position (a3) (grey outline). Circled residues were designed to contribute to helix capping. Shaded residues were designed for the introduction of restriction sites. Other residues are from c-Jun (LibA) or c-Fos (LibB). Arrows indicate putative e/g-interactions.

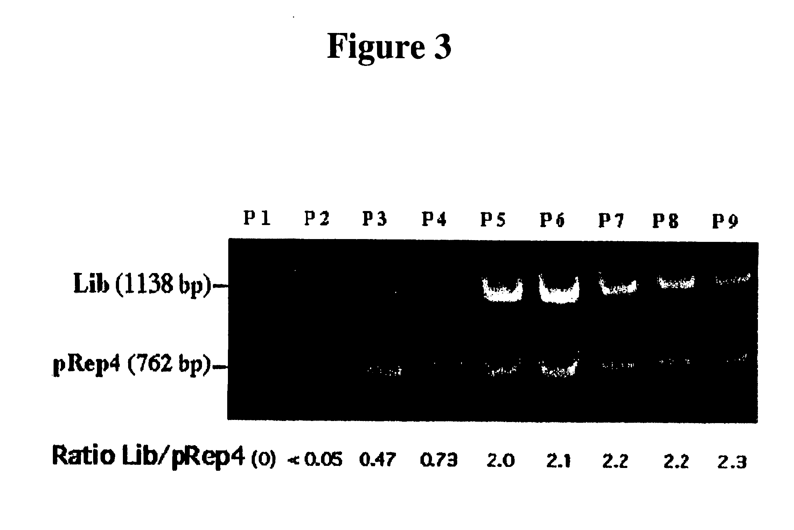

**Figure 3****:** Efficiency of competition in a model selection. The selection was set up by mixing known numbers of cells expressing either GCN4-DHFR[1]/GCN4-DHFR[2:I114A] fusions or one of 7 LibA-DHFR[1]/LibB-DHFR[2:I114A] pairs previously selected by single-step selection. The starting ratio was 2.9 x 10⁴: 1(GCN4 to Lib). Competition selection was undertaken as described in Figure 1C, and in the Experimental Protocol. The appearance of the library pairs in the pool was monitored by restriction analysis. A PvuII fragment (1138 bp) is unique to the LibB sequence of the LibB-DHFR[2] plasmid, while another (762 bp) is from pRep4 (repressor plasmid) and remains approximately constant. The bands were quantitated using the NIH Image gel analysis function to calculate the ratio of LibB/pRep4 (indicated below each lane).

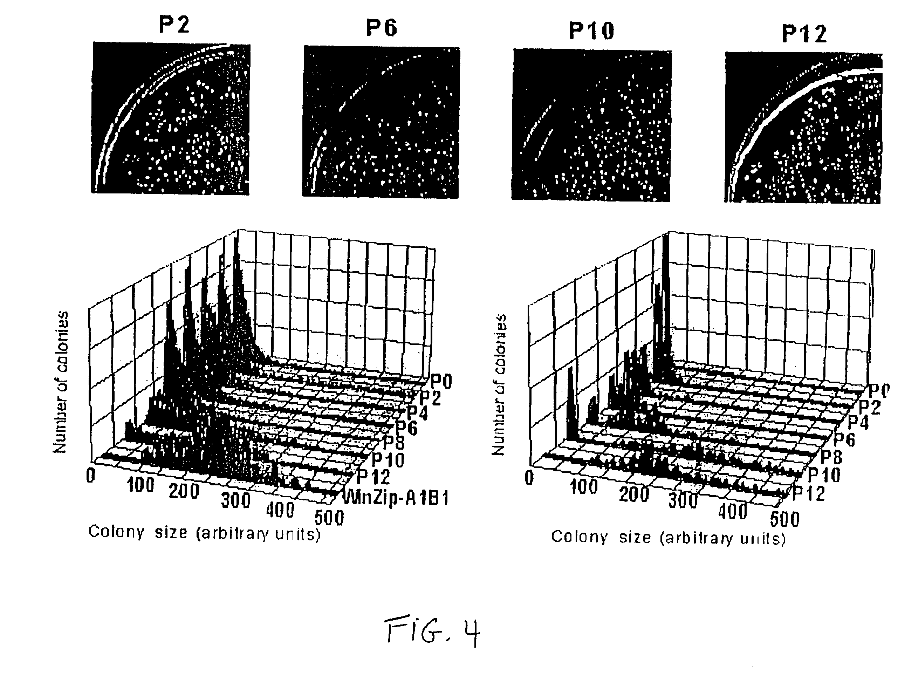

**Figure 4****:** Competition selection and chain shuffling. (A) Approximately 1.42 x 10⁴ clones resulting from single-step, I114A-mutant selection were pooled (=PO) and competition selection was undertaken as described in Figure 1C, and in the Experimental Protocol. At each passage, some cells were plated and colony sizes were quantitated. (B) Quantitation of the colony sizes from (A). For comparative purposes, quantitation of colony sizes of cells transformed with DNA of WinZip-A1B1 (but not passaged in liquid culture) is shown. (C) Quantitation of the colony sizes from passages of the chain shuffling experiment: WinZip-B1-DHFR[2:I114A] + LibA-DHFR[1]. In (B) and (C) the numbers of colonies were normalized such that passages could be directly compared.

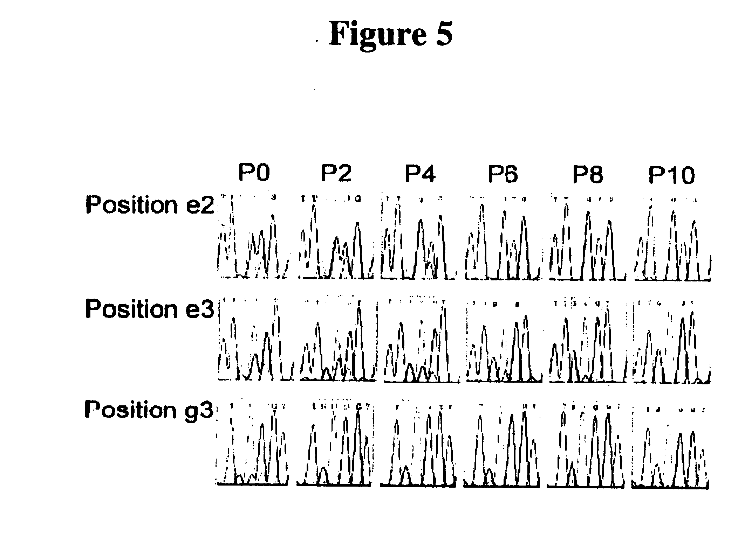

**Figure 5****:** Sequencing profile of pools from passages of the chain shuffling WinZip-B1-DHFR[2:I114A] + LibA-DHFR[1]. Representative semi-randomized positions (see Fig. 2) were taken from a single competition experiment, such that the selection rates can be directly compared. The ratio of the individual triplet codons (central three nucleotides of each frame) was visually estimated (CAG = Gln; GAG = Glu; AAG = Lys; CGT = Arg; the equimolar random mix of the 4 codons results in the predominance of C at the first position, A at the second and G at the third). Mixed positions are marked by (NNN), positions where a single codon is dominant (≥50%) are marked in lower case and those where the codon is clear (≥90%) are marked in upper case. For passages 0, 2 and 8, two independent sequencing reactions were performed, which yielded identical results.

### SUMMARY OF THE INTENTION

The present invention is directed to a method for identifying an interacting set of molecules, comprising:
(a) generating complementary first and second fragments of a reporter molecule which has a directly or indirectly detectable activity, said detectable activity being reconstituted upon association of a first fragment with a second fragment;
(b) coupling said first fragments to members of a first library of molecules to yield a first set of products;
(c) coupling said second fragments to members of a second library of molecules to yield a second set of products;
(d) mixing said first set of products with said second set of products;
(e) directly or indirectly testing for reconstitution of said detectable activity; and
(f) identifying library members whose interaction results in said detectable activity;
wherein said library members are selected from said first and second libraries of molecules;
wherein different interacting sets are introduced into separate host cell populations; and
wherein identifying the library members comprises identifying an interacting set that provides its host cells with a growth advantage relative to cells containing a different interacting set, wherein said interacting set of molecules, said first library of molecules and said second library of molecules are selected from the group of peptide and protein molecules; and
wherein said directly or indirectly detectable activity of said reporter molecule is dihydrofolate reductase activity.

Within the context of the present invention a panel is a library, which is a molecularly generated collection of compounds that structurally or functionally differ from one another.

### DESCRIPTION OF THE PREFERRED EMBODIMENTS

We investigated a large-scale selection of dimerizing leucine zipper pairs from two designed semi-randomized libraries. These libraries are a hybrid between GCN4 and c-Jun/c-Fos (Fig. 2), where the central, core "a"-position (a₃) was randomized to either N or V, with equal probability, and the recurring "e" and "g" positions were randomized to Gln (neutral), Glu (acidic), Arg or Lys (basic), each with 25% probability. This was achieved by synthesizing oligonucleotides containing synthetic codon building blocks¹⁴; the details of the design will be published elsewhere (K.M. Arndt, J.N. Pelletier, K.M. Müller, T. Alber, S.W. Michnick and A. Plückthun, submitted). This library design allowed a number of complex optimization problems to be solved simultaneously by biological selection. At the core a-position the choice of V-V pairing, which confers higher thermodynamic stability to helix pairs, competes with N-N pairing, which confers specificity of parallel dimerization with a defined packing register and disfavors formation of antiparallel dimers and higher order oligomers^{15,16}. Additionally, the importance of charged residues at the "e" and "g" positions was investigated. Formation of salt bridges between these positions of opposite monomers has been crystallographically observed¹⁷ and has been proposed to contribute to the stability of dimer formation¹⁸⁻²⁰ Additionally, the avoidance of unfavorable electrostatic interactions between same-charged residues may be more important in driving stable, specific interactions and avoiding the formation of homodimers²¹. Furthermore, the energy of charged-neutral interactions has been shown to be similar to that of charged e/g-pairs in several cases^{18,21}. Other factors, such as contribution of e/g-residues to helix propensity and helix dipole stabilization add to the difficulty of predicting the optimal e/g-pairs in dimerization even in simple model systems. Although a restricted number of positions were semi-randomized here (4 residue types at 8 positions and 2 residue types at 1 position, resulting in 1.31 x 10⁵ variants per library, and 1.7 x 10¹⁰ library-*vs*-library combinations), a problem of extraordinary complexity was generated, making predictions of the outcome very challenging. Resolution of this problem required a powerful selection strategy, which could be rapidly performed and analyzed; to our knowledge the DHFR fragment complementation system is currently the only strategy amenable to this.

Single-step selection. The semi-randomized designed leucine zipper libraries were subcloned into the appropriate vector harboring either mDHFR fragment (Fig. 1A and Experimental Protocol). As a first step in selection of heterodimerizing leucine zippers, a single-step selection was undertaken, using the wild-type mDHFR fragments, by cotransforming the libraries LibA-DHFR[1] and LibB-DHFR[2] and plating on selective media (Fig. 1B). This strategy applies only a low stringency of selection to the potential pairs, thus many library combinations were expected to be selected. Approximately 1.7% of the resulting ampicillin-resistant cells were doubly transformed, harboring (at least) one plasmid from each library when using 5 ng of each DNA, or 8% were doubly transformed when using 20 ng of each DNA, as seen from control transformations (calculated as described in the Experimental Protocol; data not shown). Of the doubly transformed cells which harbor no mutations or frame-shifts, approximately 35% formed colonies under selective conditions (**Table** 1). This result immediately demonstrates that even with relatively low stringency of selection, only a fraction of the possible combinations of the two libraries allows zipper heterodimerization leading to efficient mDHFR reassembly.

**TABLE 1: Stringency of the selection steps: selection factors**

| **Single-step selection** | | | Selection Factor^{a} |
|---|---|---|---|
| Wt mDHFR fragments (5 or 20 ng) | | | 2.8 |
| 1114A mDHFR fragments (5 or 20 ng) | | | 1.4 x 10² |

| **Competition selection** | Initial Diversity | Frequency of dominant | Selection factor^{a} |
|---|---|---|---|
| | | Pair at P12° | |
| Competition (I114A) | 3.9 x 10⁶ | WinZipA 1-B 1:18/22(82%) | 3.2 x 10⁶ |
| Shuffling: WinZip-A1 + LibB-DHFR[2:11 14A] | 1.3 x 10⁵ | WinZipA1-B2:4/6 (67%) | 8.7 x 10⁴ |
| Suffling: WinZip-B1 + LibA-DHFR[1] | 1.3 x 10⁵ | WinZipA2-B 1: 4/4 (100%) | >1.3 x 10⁵ |

| | | | |
|---|---|---|---|
| ^{a} The selection factor in single-step selection is defined as the number of cotransformed cells plated (**considering only the 50% which give combinations with no mutations or frame-shifts**), divided by the number of colonies surviving under selective conditions (see Results); average of 2 independent experiments. This value must be calculated at low DNA concentrations (≤ 20 ng of each DNA) since the multiple cotransformations occuring at high DNA concentrations mask the actual selection factor. ^{b}P12 is the 12^{th} round of serial cell passaging and competitive growth. The selection factor in competition selection is defined as the proportion of the dominant pair multiplied by the sequence diversity it was selected from. | | | |

Fourteen colonies resulting from two independent cotransformations were picked and the sequences encoding the zippers were determined. Even under these low stringency conditions there exist important sequence biases in these sequences relative to the unselected ones (Fig. 2B). A reduction in same-charged e/g-pairs from 31.3% (unselected) to 19% (selected) and an increase in opposite-charged pairs from 25% (unselected) to 31 % (selected) were seen. As well, a strong enrichment of N-N pairing at the core α-position (25% unselected vs 57% selected) was observed. The characteristics that have been enriched are consistent with the selection of stable leucine zipper heterodimers. **Use of the mDHFR Ile114Ala mutation.** We repeated the single-step selection, using the Ilel14Ala mutant of mDHFR^{4,5}, in order to increase the stringency of selection. We reasoned that only library partners that form the most stable heterodimers can compensate for the reduced ability of the mDHFR(Ile114Ala) fragments to fold into active enzyme, resulting in higher enzyme activity and growth rates. When bacteria were cotransformed with LibA-DHFR[1] and LibB-DHFR[2:I114A], we observed a 50-fold decrease in the number of colonies upon selective plating compared to the wild-type DHFR fragments (Table 1). Twenty-five colonies were picked from 3 independent cotransformations and the DNA sequences were analyzed. The increase in selectivity was concomitant with an extremely strong selection for N-N pairing at the core a-position (92%: Fig. 2B), illustrating that the specificity of in-register parallel alignment provided by N-N pairing is more highly favored under these in-vivo selection conditions than the higher stability afforded by V-V pairing. Reassembly of mDHFR from its fragments requires that in the final structure. the two fragment *N*-termini be brought close enough together to allow native-like refolding of DHFR (Fig. 1)^{5,22}. The peptide linkers that connect the library sequences to the DHFR fragments must be sufficiently flexible to allow DHFR to fold from its fragments, but not so long that any C-terminal to N-terminal orientation of the final folded leucine zipper would be allowed. As a result of this structural requirement, parallel in-register heterodimerization of the library peptides is the only configuration possible. Other biases in these sequences were also more pronounced than with the wt DHFR fragments (Fig. 2B). In particular, an additional increase in opposite-charged e/g-pairs from 31% to 37% was seen. In one case, a point-mutation resulted in a single clone (1/25) with a V-T pair at the core α-position.

**Competition selection: Efficiency of selection.** To further increase the selection pressure, we applied the principle of competition selection. We reasoned that, among selected zipper pairs, those which result in more stable heterodimerization will allow the most efficient enzyme reconstitution, leading to higher DHFR activity. If DHFR activity is limiting for growth, the higher activity should result in more rapid bacterial propagation, hence these cells would become enriched in a pool. Thereby, after sequential rounds of growth-competition, subtle differences in growth rate can be amplified, increasing the stringency of selection relative to the single-step selection.

To determine the rate at which competition can enrich for particular partner pairs, we first set up a model competition with a limited number of clones as described in Figure 1C. The initial cell mixture (P0) contained known amounts of viable cells expressing either GCN4-DHFR[1]/GCN4-DHFR[2:I114A] or one of seven LibA-DHFR[1]/LibB-DHFR[2:I114A] pairs previously obtained in a single-step selection of those libraries, mixed at a ratio of 2.9 x10⁴ : 1 (GCN4 : library clones). Productive association of the homodimeric GCN4 pair should occur only 50% of the time *versus* up to 100% for heterodimerizing library clones, thus is disadvantaged. Within 3 passages, the library pairs were already visibly enriched (Fig. 3), and after 5 passages the measured ratio between a restriction fragment indicative of the library and a constant fragment from the repressor plasmid had reached its maximium, showing that enrichment was maximal. Colonies resulting from passage 9 (P9) were sequenced. No GCN4 leucine zippers were present among 24 sequences analyzed. Therefore, enrichment of the library pairs over GCN4 by a factor of at least 24 x 2.9 x 10⁴ = 7 x 10⁵ was achieved. Four out of the 7 library clones initially present survived until P9, with varying distributions (data no shown). The experiment was also repeated at a lower starting ratio of GCN4 and the same library clones were enriched, consistent with their enrichement being truly the result of selection (and not of unrepresentative sampling). This indicated that selection among the pre-selected clones was not as rapid as that seen between pre-selected and GCN4 zippers, but that the smaller differences between the pre-selected ones can still be amplified in selection. These results demonstrate that there is a direct link between reconstitution of mDHFR and growth rate. **Competition selection for optimal pairs:** Our ultimate goal was to select for the "best" among the zipper pairs obtained by single-step selection. We obtained a large initial number of clones by cotransforming bacteria with 0.5 µg of DNA each from LibA-DHFR[1] and LibB-DHFR[2:I114A]. Approximately 50% of cells were at least doubly transformed (52% 10%, average of 2 independent control experiments, calculated as described in the Experimental Protocol). We obtained approximately 1.42 x 10⁴ clones on selective medium, which arise from a 1.4 x 10²-fold selection factor (see Table 1), and were thus selected from (1.42 x 10⁴) x (1.4 x 10²) = 2.0 x 10⁶ library-*vs*-library cotransformants. These were pooled and passaged. There was a clear increase in colony sizes with subsequent passages, indicating that faster-growing clones were taking over (Fig. 4A, B). At P12, the colonies are homogeneously large, showing similar growth rates among the clones. Twenty-two individual colonies from P12 were picked and sequenced, as well as 11 from P10 and 2 from each previous second passage. A single pair (WinZip-A1B1, composed of WinZip-A1-DHFR[1] and WinZip-B1-DHFR[2:I114A]) was identified 18/22 times (82%) in P12, 4/11 (33%) in P10, but not in previous passages (Fig. 2C). While other sequences were found in early and late passages, none was as enriched as WinZip-A1B1. In order to verify that the growth rate recorded after competition (P12) was independant of bacteria-specific factors resulting from passaging, we cotransformed DNA from a pure clone of WinZip-A1B1 into fresh bacteria. The colony size distribution is similar for P12 and for the transformants (Fig. 4B), illustrating that the growth rate is a direct product of mDHFR reconstitution directed by the WinZip-A I B I pair.

The sequence bias observed at the core-a position was yet stronger here: only N-N pairing was recorded at the core α-position. When the biases at the e/g-positions were calculated according to the occurrence of each sequence (n=37), there was no significant change in opposite charged pairing (37%), while a small increase in same-charged pairing was observed (from 23% to 26%) as a result of the two same-charged pair which occur in the predominant WinZip-A1B1 (Fig. 2B, C). However, when each unique sequence was considered only once (n=10) a further increase of opposite-charged e/g-pairing was observed.

**Chain shuffling of the WinZip-A1B1 sequences.** In the above experiment, WinZip-A1B1 was selected from a sample representing 2.0 x 10⁶ library-*vs*-library cotransformants. As the theoretical library-*vs*-library diversity is (1.31 x 10⁵)² = 1.72 x 10¹⁰, approximately 0.01 % of the library-*vs*-library space was sampled. However, we obtained a very high coverage of either single library (theoretical complexity of 1.31 x 10⁵), where the probability of all members being present at least once is P=0.973. Thus, each polypeptide sampled only a small portion of the opposite library (2.0 x 10⁶ / 1.31 x 10⁵ = 15.4 polypeptides of the other library with P=0.999, assuming equal transformation rates for both libraries) and it is likely that better combinations for the WinZip-A1B1 peptides may be found. Using WinZip-A1B1 as a partially optimized starting point, we combined each of the two WinZip-A1B1 polypeptides with the opposite library (WinZip-A1-DHFR[1] + LibB-DHFR[2:I114A] and WinZip-B1-DHFR[2:I114A] + LibA-DHFR[1]). Single-step selection yielded pre-selected pools for either competition. In both cases, the library (1.3 x 10⁵) was over-represented by a factor of 24 and 14, respectively, and the probability that all members were present at least once as partners of the "constant" peptide is P>0.999 and 0.882, respectively. With passages of selection competition, a clear increase in colony sizes was again observed, indicating that faster-growing clones were taking over (Fig. 4C).

At P0 and each second passage, DNA from the entire pool of cells was sequenced in order to follow the rate of evolution of each library against a constant partner. Figure 5 illustrates the results from representative semi-randomized positions. It is clear that the rate of selection is not constant at all positions: some positions showed a dominant residue (≥50%) already at P4 and clear selection (≥90%) at P6 (see position e2) while others remained mixed (<50%) until P6 and became clear only at P10 (see position g3). This was observed in both selections. The sequences from individual colonies were analyzed. In both selections, a predominant clone was identified (Table 1 and Fig. 2C), which is similar, but not identical, to the originally selected WinZip-A1B1 pair. The selection of the predominant clone WinZipA2B1 (selection of LibA-DHFR[1] against WinZip-B1-DHFR[2:I114A]) was achieved before P10, as P10 (4 clones analyzed) and P12 (4 clones analyzed) revealed only this clone. The selection of the predominant clone WinZipA1B2 (selection of LibB-DHFR[2:I114A] against WinZip-A1-DHFR[1]) was clear but not complete after 12 passages, as it was identified 4/6 times in P12 and 3/5 times in P10.

During the multiple passages performed in competition selection, the spontaneous acquisition of trimethoprim resistance by the *E.* coli DHFR could in principle lead to a "false-positive" result, where survival would be independent of the mDHFR fragment complementation. While we observed such a phenotype on one occasion at a rate of approximately 1 resistant clone per 2 x 10⁸ bacteria in single-step selection, we never observed this in clones resulting from competition selection, although up to 10¹² cells were used during each competition. Thus this phenotype does not interfere with the selection process.

We sequenced the regions N- and C-terminal to all zipper pairs obtained, including the promoter region and part of the mDHFR-fragment coding sequence (including residue 114). As well, the entire mDHFR fragment-coding sequence was verified in all WinZip clones. In no case was a mutation, rearrangement or a recombination of any constant portion of the constructs observed. In addition, all clones were subjected to restriction analysis, and showed normal restriction patterns (data not shown). As in all *in vivo* strategies based on fusion proteins, we cannot preclude that the selected zippers could induce folding of mDHFR from its fragments or stabilize mDHFR through interactions of the leucine zipper with either the peptide linkers or with one or both of the DHFR fragments. However, the strong selection biases we observe, particularly the perfect selection for N-N pairing under conditions of high stringency but also of complementary e-g pairings, support our hypothesis that selection is determined by heterodimerizing leucine zipper-forming peptides.

As shown above, applicant's have applied the *in-vivo* mDHFR-fragment complementation assay to select stably interacting partners in a library-*vs*-library screen for heterodimerizing leucine zippers. Selection was successful both in single-step, and in competition assays. Many combinations of the two libraries were expected to form heterodimers, albeit of varying stability. The 2.8-fold selection factor observed in single-step selection using the wild-type mDHFR fragments is consistent with the expectation that many of the combinations should result in functional heterodimers, since 9 of the 10 a- and d-positions that define the hydrophobic core were invariant. Use of the I114A-mutant of mDHFR increased the stringency of selection 50-fold, and competition selection allowed amplification of the most successful pairs from this pool. The sequence biases observed indicate that selection favored N-N pairing very strongly over V-V pairing in the hydrophobic core, consistent with selection for specificity of parallel, in-register dimerization. This in-register alignment allows the direct comparison of the selected zippers as all helices are forced, by the N-N pair, to assume a parallel orientation, juxtaposing the same e- and g-residues in all selected library members. Opposite-charged e/g-pairs were generally, but not exclusively favored, suggesting that building stable zippers with good in-vivo performance is more complex than simply designing opposite-charged pairs. The increasing colony sizes observed during competition are consistent with selection based on higher levels of reconstituted mDHFR activity. Our results suggest that competition selection could be undertaken as a continuous culture in automated protein evolution schemes, and should be robust as we have observed no genetic instabilities. We efficiently isolated a predominant individual clone (WinZip-A1B1) from approximately 2 x 10⁶ individual combinations, taken from a 10¹⁰ combinatorial space. The biophysical characterization of this novel leucine zipper confirms that it is stable and strongly heterodimerizing (K.M. Arndt *et al.,* submitted). Taken with the observed sequence biases and success in growth competition, it appears that there is a direct link between stability of zipper interaction and success in the selection process. To our knowledge, this is the first demonstration of a large-scale library-*vs*-library selection procedure for the optimization of protein-protein interactions.

We obtained the WinZip-A1B 1 pair from a partial sampling of the combinatorial space. In order to determine if WinZip-A1B could be improved, we performed a "chain shuffling" experiment. As each library was very well represented in this second selection, the best match within each library for the given partner should have been found. In both shuffling competitions, the population gradually converged to a predominant clone (WinZip-1A-WinZip-2 and WinZip-2A -WinZip-1B), which is similar, but not identical, to the originally selected WinZip-A1B1 pair. This indicates that the partial sampling of the 10¹⁰ sequence space yielded a good, though not optimal product (WinZip-A1B1), which was easily further improved by shuffling. The residues selected at the semi-randomized positions of these novel leucine zipper pairs differ somewhat from known natural zippers or designed zippers, yet they behaved best in this system. It is likely that other factors, such as helix-propensity and interactions of the charges with the helix dipole also contribute to the stability. This underscores the advantages of semi-rational design accompanied by selection in an appropriate in-vivo setting.

An important insight that can be gained from varying a library against a constant partner is how the selection occurs. Two scenarios can be envisaged. In the first, the selective pressure is not equivalent at all randomized positions, such that the rate of selection is rapid at certain positions while other positions remain semi-randomized longer. In the second scenario, selective pressure is applied against each polypeptide as a whole, perhaps because the identity of a residue at one position constrains the choice at other positions within the same helix. This would result in a population where the rate of selection is independent of positional pressures arising from the partner. The results we obtain are clearly consistent with the first scenario. In particular, the core α-position showed the fastest rate of selection. While an in-depth analysis of the positional rate of selection is beyond the scope of this paper and will be presented in conjunction with the biophysical characterization of the optimized WinZip peptides (K.M. Arndt, J.N. Pelletier, K.M. Müller, S.W. Michnick, T. Alber and A. Plückthun, manuscript in preparation), we believe that the rate of selection is a reflection of the contribution of a residue at a given position, to the global coiled-coil interaction.

The DHFR-fragment complementation assay has important advantages over selection techniques such as phage display in that it is possible to perform library-*vs*-library screening. Furthermore, the interactions occur *in vivo* rather than *in vitro,* which is important where *in vivo* performance is a quality of interest. Among *in vivo*-based selection strategies, it has the principal advantages of being fast and simple to execute and of having a direct link between protein-protein interactions and cell propagation, thus allowing selection rather than screening. The λ-repressor dimerization strategy²³ has been applied to selection schemes similar to that presented here^{15,24}, but this system requires complex experimental analysis and interpretation in order to distinguish homo- from heterodimerization, and again from formation of higher-order oligomers, thus precluding thorough characterization of a large number of pairs. While library-*vs*-library screening should be possible using the selectively-infective phage (SIP) system^{25,26}, this has not yet been demonstrated. The yeast two-hybrid strategy has been very successfully applied, among other tasks, to the process of systematically mapping interactions in yeast by successive rounds against single baits^{27,28}. Although there is no *a priori* reason for not performing library-*vs*-library screens with the two-hybrid strategy, with the possible exception of less efficient transformation than of *E. coli,* this has not been reported to date. In addition, we have previously demonstrated⁵ that the DHFR-fragment complementation assay can be used as a "three-hybrid" assay²⁹, where a third partner is required to mediate the protein-protein interaction. In the case we presented, the third partner was a small ligand. It should be possible to extend the assay such that the third partner is either an expressed protein or a specific RNA. This library-*vs-*library strategy will be an invaluable tool in defining networks of interacting polypeptides in functional genomics. The current limitation of the strategy is the transformation efficiency of the *E. coli* strain used. Possible improvements include the use of a bacterial host with better transformation yields than BL21, or a more efficient transformation strategy.

In conclusion, the DHFR fragment complementation assay has been used in a selection strategy for library-*vs*-library screening of optimally-interacting leucine zippers, in an *in-vivo* context. The selected, heterodimerizing peptides should be appropriate for direct use in efficient protein heterodimerization strategies. The possibility of now screening two libraries against each other will allow for a much deeper exploration of complementary surfaces than is possible using a single library approach. By using a semi-rational approach for screening a large number of interacting partners in a "two-dimensional" fashion, many factors contributing to interaction specificity will be identifiable.

### EXAMPLES

All reagents used were of the highest available purity. Sequencing was carried out either by cycle sequencing with fluorescence labeling (MWG-Biotech) using a LiCor detection system or by automated sequencing with an ABI sequencer. Restriction endonucleases and DNA modifying enzymes were from Pharmacia and New England Biolabs. *E. coli* strain XL1-Blue (Stratagene) was used for subcloning and propagation of the libraries. *E. coli* strain BL21 harboring the *lacI^{q}* plasmid pRep4 (Qiagen) was cotransformed with the appropriate DNA constructs for the survival assays.

### EXAMPLE 1

**Constructs for DHFR fragment complementation:** The DNA constructs encoding the N-terminal (1-107) and *C*-terminal (108-186) mDHFR fragments have been previously described⁵. Briefly, each fragment was amplified by PCR with appropriate unique flanking restriction sites and subcloned into a bacterial expression vector (pQE-32 from Qiagen). Each plasmid encodes an N-terminal hexahistidine tag, followed by a designed flexible linker and the appropriate DHFR fragment. Unique restriction sites between the hexahistidine tag and the flexible linker allow subcloning of the desired library. After subcloning, the resulting linker between either library and DHFR fragment was: A(SGTS)₂STSSGI for LibA and SEA(SGTS)₂STS for LibB. The design of the semi-randomized libraries is illustrated in Figure 2 and will be described in detail elsewhere (K.M. Arndt *et al*.*,* submitted). Both libraries were produced using triplet-encoding oligonucleotides¹⁴ and amplified by PCR, using primers carrying the appropriate unique restriction sites at each terminus, and the digested, gel purified products were ligated to the appropriate vector (Fig 1). To achieve maximal library representation, the ligation mixes were individually electroporated into XL1-Blue cells and selected with ampicillin on rich medium (LB). A 2- to 7-fold over-representation of each library was obtained. The resulting colonies were pooled and the plasmid DNA purified such that supercoiled plasmid DNA was obtained for cotransformation. In order to verify that the library populations encode the designed amino acids with the expected frequency, single clones from each library were randomly picked and sequenced before selection. No statistically significant biases were detected. Seventy to 80% of each library had no mutations or frame-shifts, and thus the library-*vs*-library combination yielded approximately 50% correct sequence combinations. In cotransformations, the occurrence of double transformation was calculated as the number of colonies growing under selective pressure with trimethoprim (described below) divided by the number growing in the absence, when cotransformed with equal amounts of each DNA of a given, pre-selected pair.

### EXAMPLE 2

Selection: Selective pressure for DHFR was maintained throughout all steps by inhibiting the bacterial DHFR with trimethoprim (1 µg/ml) in minimal medium. Ampicillin and kanamycin (100 µg/ml and 50 µg/ml, respectively) were also included in all steps to retain the library plasmids and the *lacI^{q}* repressor-encoding plasmid (pRep4), respectively. Expression of the proteins was induced with 1 mM IPTG. When selecting on solid medium, growth was allowed for 45 hrs at 37°C. When selecting in liquid medium, the starting O.D. (600 nm) was either 0.0005 or 0.0001. Cells were propagated either in Erlenmeyer flasks or in a 10 liter New Brunswick fermentor, depending on the volume required to ensure adequate representation of all clones present, at 37°C with shaking, or stirring at 250 RPM. After 10 to 24 hrs. O.D. (600 nm) reached 0.2 to 1.0 and cells were harvested. In competition selections, liquid culture was directly used to inoculate the next passage. We used BL21 cells with a transformation efficiency of no less than 5 x 10⁷ transformants per µg of DNA using 200 pg of DNA, or 2 x 10⁷ transformants per µg using 500 ng of DNA. In cotransformations, the occurrence of double transformation was calculated as the number of colonies growing under selective pressure with trimethoprim divided by the number growing in the absence, when cotransformed with equal amounts of each DNA of a given, pre-selected pair.

### EXMAPLE 3

**Competition selection:** When it was necessary to control precisely the starting number of cells in a competition, the number of viable cells in the starter cultures was quantitated as follows. The appropriate clones were propagated in liquid media under selective conditions and dilute aliquots were frozen at -80°C with 15% glycerol. One aliquot for each clone was thawed and plated under selective conditions, and the colonies counted after 45 hrs. The volume of cells to use for P0 was then calculated, such that each clone should be over-represented by a factor of at least 2000. Colony sizes (in Fig. 4) were evaluated using the NIH Image Particle Analysis Facility.

### EXAMPLE 4

**Chain shuffling:** DNA from the WinZip-A1B1 clone was isolated and retransformed into bacteria in order to obtain clones carrying either plasmid WinZip-A1-DHFR[1] or WinZip-B1-DHFR[2:I114A]. A pure clone (for each) was electroporated with the appropriate library. Library representation was calculated by comparison with control transformations of the same cells with DNA from the other WinZip-A1B 1 polypeptide (calculated as the number of colonies growing in the presence of trimethoprim divided by the number growing in the absence). Single-step and competition selection were undertaken as described above. It should be noted that cotransformation of bacteria at high DNA concentrations (0.5 µg per library) can lead to multiple plasmid transformation, where many survivors harbor more than one of either library sequence (data not shown). However, in no case was more than one sequence pair identified per clone after any competition selection, suggesting that multiply transformed cells retained only the pair of plasmids optimal for survival throughout the competition selection.

### REFERENCES

1. Fields, S. & Song, O. 1989. A novel genetic system to detect protein-protein interactions. Nature 340, 245-246.
2. Chien, C.T., Bartel, P.L., Sternglanz, R. & Fields, S. 1991. The two-hybrid system: a method to identify and clone genes for proteins that interact with a protein of interest. Proc. Natl. Acad. Sci. U S A 88, 9578-9582.
3. Smith, G.P. 1985. Filamentous fusion phage: novel expression vectors that display cloned antigens on the virion surface. Science 228, 1315-1317.
4. Pelletier, J.N., Remy, I. and Michnick, S. W. 1998. Protein-fragment complementation assays: a general strategy for the in vivo detection of protein-protein interactions. J. Biomol. Tech., http://www.abrf.org/JBT/Articles/JBT0012/jbt0012.html.
5. Pelletier, J.N., Campbell-Valois, F.X. & Michnick, S.W. 1998. Oligomerization Domain-Directed Reassembly of Active Dihydrofolate Reductase From Rationally Designed Fragments.. Proc. Natl. Acad. Sci. U S A 95, 12141-12146.
6. Remy. I. & Michnick. S.W. 1999. Clonal Selection and In Vivo Quantitation of Protein Interactions with Protein Fragment Complementation Assays. Proc Natl Acad Sci U S A 96, 5394-5399.
7. Remy, I., I. A. Wilson, and S. W. Michnick 1999. Erythropoietin receptor activation by a ligand-induced conformation change. Science 283, 990-993.
8. Sydor, J.R., Engelhard, M., Wittinghofer, A.. Goody, R.S. & Herrmann, C. 1998. Transient kinetic studies on the interaction of Ras and the Ras-binding domain of c-Raf 1 reveal rapid equilibration of the complex. Biochemistry 37, 14292-14299.
9. Chen, J., Zheng, X.F., Brown, E.J. & Schreiber, S.L. 1995. Identification of an 11-kDa FKBP12-rapamvcin-binding domain within the 289-kDa FKBP12-rapamycin-associated protein and characterization of a critical serine residue. Proc. Natl. Acad. Sci. U S A 92, 4947-4951.
10. O'Shea, E.K., Lumb, K.J. & Kim, P.S. 1993. Peptide 'velcro': Design of a heterodimeric coiled coil. Current Biology 3, 658-667.
11. Jelesarov, I. & Bosshard, H.R. 1996. Thermodynamic characterization of the coupled folding and association of heterodimeric coiled coils (leucine zippers). J. Mol. Biol. 263, 344-358.
12. Zhou, N.E., Kay, C.M. & Hodges, R.S. 1994. The role of interhelical ionic interactions in controlling protein folding and stability. De novo designed synthetic two-stranded alpha-helical coiled-coils. J. Mol. Biol. 237, 500-512.
13. Müller, K.M., Arndt, K.M., Strittmatter, W. & Plückthun, A. 1998. The first constant domain (CH1 and CL) of an antibody used as heterodimerization domain for bispecific miniantibodies. FEBS Lett. 422, 259-264.
14. Virnekas, B., Ge. L., Plückthun. A., Schneider. K.C., Wellnhofer, G. & Moroney, S.E. 1994. Trinucleotide phosphoramidites: ideal reagents for the synthesis of mixed oligonucleotides for random mutagenesis. Nucleic Acids Res. 22, 5600-5607.
15. Zeng, X., Herndon, A.M. & Hu, J.C. 1997. Buried asparagines determine the dimerization specificities of leucine zipper mutants. Proc. Natl. Acad. Sci. U S A 94, 3673-3678.
16. Lumb, K.J. & Kim, P.S. 1995. A buried polar interaction imparts structural uniqueness in a designed heterodimeric coiled coil. Biochemistry 34, 8642-8648.
17. O'Shea, E.K., Klemm, J.D., Kim, P.S. & Alber, T. 1991. X-ray structure of the GCN4 leucine zipper, a two-stranded, parallel coiled coil. Science 254, 539-544.
18. Zhou. N.E., Kay, C.M. & Hodges, R.S. 1994. The net energetic contribution of interhelical electrostatic attractions to coiled-coil stability. Protein Eng. 7, 1365-1372.
19. Monera, O.D., Kay, C.M. & Hodges, R.S. 1994. Electrostatic interactions control the parallel and antiparallel orientation of alpha-helical chains in two-stranded alpha-helical coiled-coils. Biochemistry 33, 3862-3871.
20. John, M., Briand, J.P., Granger-Schnarr, M. & Schnarr, M. 1994. Two pairs of oppositely charged amino acids from Jun and Fos confer heterodimerization to GCN4 leucine zipper. J. Biol. Chem. 269, 16247-16253.
21. Lumb, K.J. & Kim, P.S. 1995. Measurement of interhelical electrostatic interactions in the GCN4 leucine zipper. Science 268, 436-439.
22. Buchwalder, A., Szadkowski, H. & Kirschner, K. 1992. A fully active variant of dihydrofolate reductase with a circularly permuted sequence. Biochemistry 31, 1621-1630.
23. Hu, J.C., O'Shea, E.K., Kim, P.S. & Sauer, R.T. 1990. Sequence requirements for coiled-coils: analysis with lambda repressor-GCN4 leucine zipper fusions. Science 250, 1400-1403.
24. Zeng, X., Zhu, H., Lashuel, H.A. & Hu. J.C. 1997. Oligomerization properties of GCN4 leucine zipper e and g position mutants. Protein Sci. 6, 2218-2226.
25. Spada. S. & Plückthun, A. 1997. Selectively infective phage (SIP) technology: a novel method for in vivo selection of interacting protein-ligand pairs. Nat. Med. 3, 694-696.
26. Rudert. F., Woltering, C., Frisch, C., Rottenberger, C. & Ilag, L.L. 1998. A phage-based system to select multiple protein-protein interactions simultaneously from combinatorial libraries. FEBS Lett. 440, 135-140.
27. Bartel, P.L., Roecklein, J.A., SenGupta, D. & Fields, S. 1996. A protein linkage map of Escherichia coli bacteriophage T7. Nat. Genet. 12, 72-77.
28. Fromont-Racine. M., Rain, J.C. & Legrain, P. 1997. Toward a functional analysis of the yeast genome through exhaustive two-hybrid screens. Nat. Genet. 16, 277-282.
29. SenGupta, D.J., Zhang, B., Kraemer, B., Pochart, P., Fields, S. & Wickens, M. 1996. A three-hybrid system to detect RNA-protein interactions in vivo. Proc. Natl. Acad. Sci. U S A 93, 8496-8501.

## Claims

1. A method for identifying an interacting set of molecules, comprising:
(a) generating complementary first and second fragments of a reporter molecule which has a directly or indirectly detectable activity, said detectable activity being reconstituted upon association of a first fragment with a second fragment;
(b) coupling said first fragments to members of a first library of molecules to yield a first set of products;
(c) coupling said second fragments to members of a second library of molecules to yield a second set of products;
(d) mixing said first set of products with said second set of products;
(e) directly or indirectly testing for reconstitution of said detectable activity; and
(f) identifying library members whose interaction results in said detectable activity;
wherein said library members are selected from said first and second libraries of molecules;
wherein different interacting sets are introduced into separate host cell populations; and
wherein identifying the library members comprises identifying an interacting set that provides its host cells with a growth advantage relative to cells containing a different interacting set, wherein said interacting set of molecules, said first library of molecules and said second library of molecules are selected from the group of peptide and protein molecules; and
wherein said directly or indirectly detectable activity of said reporter molecule is dihydrofolate reductase activity.

2. The method of claim 1 wherein a library of molecules is a molecularly generated collection of compounds that structurally or functionally differ from one another.

3. The method of claim 1 wherein said reporter molecule is murine dihydrofolate reductase.

4. The method of any one of claims 1 to 3, wherein said first and second fragments are coupled to members of said first and second libraries of molecules, respectively, via a flexible linker.

5. The method of any one of claims 1 to 4, wherein said host cells are bacterial cells selected from the group consisting of:
(a) E. coli cells, and
(b) bacterial cells with a better transformation yield than *E. coli* strain BL21.

6. The method of claim 5 wherein a third molecule is required to mediate protein-protein interaction between the interacting set of molecules.

7. The method of claim 6 wherein said third molecule is a small ligand, a protein or a specific RNA.

8. The method of claim 1 wherein said interacting set of molecules is an interacting set of polypeptide molecules;
wherein said method comprises selecting for advantageous growth rate of said host cells in culture:
(a) wherein bacterial dihydrofolate reductase activity is inhibited during growth of cultures of said host cells, and
(b) wherein the growth rate of cultures of said host cells is dependent upon reporter molecule dihydrofolate reductase activity.

9. The method of claim 8 wherein host cells are cultured in the presence of the bacterial dihydrofolate reductase inhibitor trimetheprim.

10. A method for detecting and identifying an interacting set of molecules, comprising:
(a) generating complementary first and second fragments of a murine dihydrofolate reductase (mDHFR) reporter molecule, wherein reporter molecule dihydrofolate reductase activity is reconstituted when a first fragment associates with a complementary second fragment;
(b) coupling said first fragments to members of a first library of molecules via a flexible linker to yield a first set of products,
(c) coupling said second fragments to members of a second library of molecules via a flexible linker to yield a second set of products,
(d) introducing different interacting sets of products into separate E.coli host cell populations;
(e) culturing said E.coli host cell populations in the presence of the bacterial dihydrofolate reductase inhibitor trimetheprim, thereby selecting for reconstitution of said reporter molecule dihydrofolate reductase activity; and
(f) identifying library members whose interaction as components of interacting sets of products results in said reporter molecule dihydrofolate reductase activity, wherein said library members are selected from said first and second libraries of molecules, and wherein identifying the library members comprises identifying an interacting set of products that provides its host cells with a growth advantage relative to cells containing a different interacting set of products;
wherein, in said method, said interacting set of molecules, said first library of molecules and said second library of molecules are each selected from the group of peptide and protein molecules.

11. The method of any one of claims 8 to 10 for identifying interacting sets of molecules exhibiting the highest reporter molecule activities; said method comprising competitive metabolic selection, wherein clones obtained by selecting for advantageous growth rate of the host cells in culture are pooled and passed through sequential rounds of growth-competition selection.

12. The method of claim 11 wherein the competition selection is undertaken as a continuous culture in an automated protein evolution scheme.

13. The method of any one of claims 8 to 11 wherein said interacting set of molecules is an interacting set of coiled-coil forming peptide molecules.

14. The method of any one of claims 1 to 3 for identifying interacting sets of molecules exhibiting the highest reporter molecule activities, wherein the avidity of said first and second reporter molecule fragments for one another is decreased relative to a reference set of fragments.

15. The method of any one of claims 8 to 11, wherein said first fragment or said second fragment is a mutant DHFR fragment which comprises the mutation dihydrofolate reductase [IIe114Ala] and which prevents stable reassembly of DHFR from its first and second fragments.

16. The method of any one of claims 8 to 11 and 15, wherein said first library of molecules and said second library of molecules each comprises a library of leucine zipper-forming peptides.

17. The method of claim 16 wherein said leucine zipper-forming peptides are selected from the group consisting of peptides interacting with WinZip A1 and peptides interacting with WinZip B1.

## Patentansprüche

1. Verfahren zum Identifizieren eines interagierenden Sets von Molekülen, umfassend:
(a) Erzeugen von komplementären ersten und zweiten Fragmenten eines Reportermoleküls, das entweder eine direkt oder eine indirekt detektierbare Aktivität hat und die detektierbare Aktivität nach Assoziation eines ersten Fragments mit einem zweiten Fragment hin wiederhergestellt wird;
(b) Koppeln der ersten Fragmente an Mitglieder einer ersten Molekülbibliothek, um ein erstes Set von Produkten zu erhalten;
(c) Koppeln der zweiten Fragmente an Mitglieder einer zweiten Molekülbibliothek, um ein zweites Set von Produkten zu erhalten;
(d) Mischen des ersten Sets von Produkten mit dem zweiten Set von Produkten;
(e) direktes oder indirektes Testen auf Wiederherstellung der detektierbaren Aktivität; und
(f) Identifizieren von Bibliotheksmitgliedern, deren Interaktion in der dektierbaren Aktivität resultiert;
wobei die Bibliotheksmitglieder von der ersten und zweiten Molekülbibliothek ausgewählt werden;
wobei unterschiedliche, interagierende Sets in separate Wirtszellpopulationen eingeführt werden; und
wobei das Identifizieren der Bibliotheksmitglieder das Identifizieren eines interagierenden Sets umfasst, das seinen Wirtszellen einen Wachstumsvorteil gegenüber den Zellen, die ein anderes interagierendes Set enthalten, verschafft, wobei das interagierende Set von Molekülen, die erste Molekülbibliothek und die zweite Molekülbibliothek von der Gruppe ausgewählt werden, die aus Peptid- und Proteinmolekülen besteht; und
wobei die direkt oder indirekt detektierbare Aktivität des Reportermoleküls eine Dihydrofolatreduktaseaktivität ist.

2. Verfahren gemäß Anspruch 1, wobei eine Molekülbibliothek eine molekular erzeugte Ansammlung von Verbindungen ist, die sich strukturell und funktionell voneinander unterscheiden.

3. Verfahren gemäß Anspruch 1, wobei das Reportermolekül eine murine Dihydrofolatreduktase ist.

4. Verfahren gemäß einem der Ansprüche 1 bis 3, wobei die ersten und zweiten Fragmente an Mitglieder der ersten und zweiten Molekülbibliothek über einen flexiblen Linker gekoppelt sind.

5. Verfahren gemäß einem der Ansprüche 1 bis 4, wobei die Wirtszellen bakterielle Zellen sind, die aus der Gruppe ausgewählt werden, die sich zusammensetzt aus:
(a) *E. coli* Zellen und
(b) bakteriellen Zellen mit einer besseren Transformationsrate als der *E. coli* Stamm BL21.

6. Verfahren gemäß Anspruch 5, wobei ein drittes Molekül benötigt wird, um eine Protein-Protein-Interaktion zwischen dem interagierenden Set von Molekülen zu vermitteln.

7. Verfahren gemäß Anspruch 6, wobei das dritte Molekül ein kleiner Ligand, ein Protein oder eine spezifische RNA ist.

8. Verfahren gemäß Anspruch 1, wobei das interagierende Set von Molekülen ein interagierendes Set von Polypeptidmolekülen ist;
wobei das Verfahren das Auswählen für eine vorteilhafte Wachstumsrate der Wirtzellen in Kultur umfasst:
(a) wobei die bakterielle Dihydrofolatreduktaseaktivität während des Wachstums der Wirtszellkulturen gehemmt ist, und
(b) wobei die Wachstumsrate der Wirtszellkulturen abhängig von der Dihydrofolatreduktaseaktivität des Reportermoleküls ist.

9. Verfahren gemäß Anspruch 8, wobei die Wirtszellen in Anwesenheit des bakteriellen Dihydrofolatreduktaseaktivitätsinhibitors Trimetheprim kultiviert werden.

10. Verfahren zum Detektieren und Identifizieren eines interagierenden Sets von Molekülen, umfassend:
(a) Erzeugen von komplementären ersten und zweiten Fragmenten eines murinen Dihydrofolatreduktase (mDHFR)-Reportermoleküls, wobei die Dihydrofolatreduktaseaktivität des Reportermoleküls wiederhergestellt wird, wenn ein erstes Fragment mit einem komplementären zweiten Fragment assoziiert;
(b) Koppeln der ersten Fragmente an Mitglieder einer ersten Molekülbibliothek, über einen flexiblen Linker, um ein erstes Set von Produkten zu erhalten;
(c) Koppeln der zweiten Fragmente an Mitglieder einer zweiten Molekülbibliothek, über einen flexiblen Linker, um ein zweites Set von Produkten zu erhalten;
(d) Einführen von unterschiedlichen, interagierenden Sets von Produkten in separate *E. coli* Wirtszellpopulationen;
(e) Kultivieren der *E. coli* Wirtszellpopulationen in Anwesenheit des bakteriellen Dihydrofolatreduktaseaktivitätsinhibitors Trimetheprim und dabei Auswählen für die Wiederherstellung der Dihydrofolatreduktaseaktivität des Reportermoleküls; und
(f) Identifizieren von Bibliotheksmitgliedern, deren Interaktion als Komponenten von interagierenden Sets von Produkten in der Dihydrofolatreduktaseaktivität des Reportermoleküls resultiert, wobei die Bibliotheksmitglieder von der ersten und zweiten Molekülbibliothek ausgewählt werden und, wobei das Identifizieren der Bibliotheksmitglieder das Identifizieren eines interagierenden Sets von Produkten umfasst, das seinen Wirtszellen einen Wachstumsvorteil gegenüber den Zellen verschafft, die ein anderes interagierendes Set von Produkten enthalten;
wobei in dem Verfahren das interagierende Set von Molekülen, die erste Molekülbibliothek und die zweite Molekülbibliothek jeweils von einer Gruppe von Peptid-und Proteinmolekülen ausgewählt werden.

11. Verfahren gemäß einem der Ansprüche 8 bis 10 zum Identifizieren interagierender Sets von Molekülen, die die höchsten Reportermolekülaktivitäten aufweisen, wobei das Verfahren eine kompetitive metabolische Auswahl umfasst, in der die Klone, die durch die Auswahl für eine vorteilhafte Wachstumsrate der Wirtszellen erhalten werden, gebündelt und durch aufeinander folgende Runden von Wachstumskompetitionsauswahl geführt werden.

12. Verfahren gemäß Anspruch 11, wobei die Kompetitionsauswahl als eine kontinuierliche Kultur in einem automatisierten Proteinevolutionsschema vorgenommen wird.

13. Verfahren gemäß einem der Ansprüche 8 bis 11, wobei das interagierende Set von Molekülen ein interagierendes Set von Coiled-Coil-bildenden Peptidmolekülen ist.

14. Verfahren gemäß einem der Ansprüche 1 bis 3 zum Identifizieren interagierender Sets von Molekülen, die die höchsten Reportermolekülaktivitäten aufweisen, wobei die Avidität der ersten und zweiten Reportermolekülfragmente füreinander im Verhältnis zu einem Referenzset von Fragmenten erniedrigt ist.

15. Verfahren gemäß einem der Ansprüche 8 bis 11, wobei das erste Fragment oder das zweite Fragment ein mutiertes DHFR-Fragment ist, das die Mutation Dihydrofolatreduktase [IIe114Ala] umfasst und die den stabilen Wiederzusammmenbau von DHFR aus dessen ersten und zweiten Fragmenten verhindert.

16. Verfahren gemäß einem der Ansprüche 8 bis 11 und 15, wobei die erste Molekülbibliothek und die zweite Molekülbibliothek jeweils eine Bibliothek von Leucin-Zipper-bildenden Peptiden umfasst.

17. Verfahren gemäß Anspruch 16, wobei die Leucin-Zipper-bildenden Peptide von der Gruppe ausgewählt werden, die sich aus Peptiden, die mit WinZip A1 interagieren und aus Peptiden, die mit WinZip B1 interagieren, zusammensetzt.

## Revendications

1. Procédé pour identifier un ensemble de molécules interactives, comprenant:
(a) générer des premiers et des deuxièmes fragments complémentaires d'une molécule rapporteuse qui a une activité détectable directement ou indirectement, ladite activité détectable étant reconstituée à l'association d'un premier fragment avec un deuxième fragment;
(b) coupler lesdits premiers fragments avec des éléments d'une première librairie de molécules, pour obtenir un premier ensemble de produits;
(c) coupler lesdits deuxièmes fragments avec des éléments d'une deuxième librairie de molécules, pour obtenir un deuxième ensemble de produits;
(d) mélanger ledit premier ensemble de produits avec ledit deuxième ensemble de produits;
(e) tester directement ou indirectement la reconstitution de ladite activité détectable; et
(f) identifier les éléments de librairie dont l'interaction a pour résultat ladite activité détectable;
dans lequel lesdits éléments de librairie sont sélectionnés parmi lesdites première et deuxième librairies de molécules;
dans lequel différents ensembles interactifs sont introduits dans des populations de cellules hôtes séparées; et
dans lequel l'identification des éléments de librairie comprend l'identification d'un ensemble interactif qui confère à ses cellules hôtes un avantage de croissance par rapport aux cellules contenant un ensemble interactif différent, dans lequel ledit ensemble interactif de molécules, ladite première librairie de molécules et ladite deuxième librairie de molécules sont sélectionnés parmi le groupe de molécules de peptide et de protéine; et
dans lequel ladite activité détectable directement ou indirectement de ladite molécule rapporteuse est une activité dihydrofolate réductase.

2. Procédé selon la revendication 1, dans lequel une librairie de molécules est une collection générée de manière moléculaire de composés qui diffèrent structurellement ou fonctionnellement l'un de l'autre.

3. Procédé selon la revendication 1, dans lequel ladite molécule rapporteuse est murine dihydrofolate réductase.

4. Procédé selon l'une quelconque des revendications 1 à 3, dans lequel lesdits premiers et deuxièmes fragments sont couplés avec des éléments respectivement desdites première et deuxième librairies de molécules, via un lieur flexible.

5. Procédé selon l'une quelconque des revendications 1 à 4, dans lequel lesdites cellules hôtes sont des cellules bactériennes sélectionnées parmi le groupe composé de :
(a) cellules *E. coli,* et
(b) cellules bactériennes avec un meilleur rendement de transformation que la lignée *E. coli* BL21*.*

6. Procédé selon la revendication 5, dans lequel une troisième molécule est requise pour intervenir dans l'interaction protéine-protéine entre l'ensemble interactif de molécules.

7. Procédé selon la revendication 6, dans lequel ladite troisième molécule est un petit ligand, une protéine ou un ARN spécifique.

8. Procédé selon la revendication 1, dans lequel ledit ensemble interactif de molécules est un ensemble interactif de molécules de polypeptide;
dans lequel ledit procédé comprend la sélection d'un taux de croissance avantageux desdites cellules hôtes dans la culture:
(a) où l'activité dihydrofolate réductase bactérienne est inhibée pendant la croissance de cultures desdites cellules hôtes, et
(b) où le taux de croissance de cultures desdites cellules hôtes est fonction de l'activité dihydrofolate réductase de la molécule rapporteuse.

9. Procédé selon la revendication 8, dans lequel les cellules hôtes sont cultivées en présence de triméthéprime inhibiteur de dihydrofolate réductase bactérienne.

10. Procédé pour détecter et identifier un ensemble interactif de molécules, comprenant:
(a) générer des premiers et secondaires fragments complémentaires d'une molécule rapporteuse de murine dihydrofolate réductase (mDHFR), où l'activité de dihydrofolate reductase de la molécule rapporteuse est reconstituée lorsqu'un premier fragment s'associe avec un deuxième fragment complémentaire;
(b) coupler lesdits premiers fragments à des éléments d'une première librairie de molécules via un lieur flexible, pour obtenir un premier ensemble de produits,
(c) coupler lesdits deuxièmes fragments à des éléments d'une deuxième librairie de molécules via un lieur flexible, pour obtenir un deuxième ensemble de produits,
(d) introduire différents ensembles interactifs de produits dans des populations de cellules hôtes *E. coli* séparées;
(e) cultiver lesdites populations de cellules hôtes *E. coli* en présence de triméthéprime inhibiteur de dihydrofolate réductase bactérienne, en sélectionnant la reconstitution de ladite activité dihydrofolate réductase de la molécule rapporteuse; et
(f) identifier les éléments de librairie dont l'interaction comme composants d'ensembles interactifs de produits a pour résultat ladite activité dihydrofolate réductase de la molécule rapporteuse, où lesdits éléments de librairie sont sélectionnés parmi lesdites première et deuxième librairies de molécules, et où l'identification des éléments de librairie comprend l'identification d'un ensemble interactif de produits qui confèrent à ses cellules hôtes un avantage de croissance par rapport aux cellules contenant un ensemble interactif différent de produits;
dans lequel, dans ledit procédé, ledit ensemble interactif de molécules, ladite première librairie de molécules et ladite deuxième librairie de molécules sont sélectionnés, chacun, parmi le groupe de molécules de peptide et protéine.

11. Procédé selon l'une quelconque parmi les revendications 8 à 10 pour identifier des ensembles interactifs de molécules présentant les plus grandes activités de molécule rapporteuse; ledit procédé comprenant une sélection métabolique compétitive, dans lequel les clones obtenus en sélectionnant un taux de croissance avantageux des cellules hôtes dans la culture sont regroupés et passés par des tours séquentiels de sélection de compétition de croissance.

12. Procédé selon la revendication 11, dans lequel la sélection de compétition est effectuée comme culture continue dans un schéma d'évolution de protéine automatisé.

13. Procédé selon l'une quelconque parmi les revendications 8 à 11, dans lequel ledit ensemble interactif de molécules est un ensemble interactif de molécules de peptide en spirale - formant une spirale.

14. Procédé selon l'une quelconque parmi les revendications 1 à 3 pour identifier des ensembles interactifs de molécules présentant les plus grandes activités de molécule rapporteuse, où l'avidité desdits premiers et deuxièmes fragments de molécule rapporteuse l'un pour l'autre est diminuée par rapport à un ensemble de fragments de référence.

15. Procédé selon l'une quelconque parmi les revendications 8 à 11, dans lequel ledit premier fragment ou ledit deuxième fragment est un fragment OHFR mutant qui comprend dihydrofolate réductase de mutation [Ile114Ala] et qui empêche le réassemblage stable de DHFR à partir de ses premier et deuxième fragments.

16. Procédé selon l'une quelconque parmi les revendications 8 à 11 et 15, dans lequel ladite première librairie de molécules et ladite deuxième librairie de molécules comprennent, chacune, une librairie de peptides formant un zipper de leucine.

17. Procédé selon la revendication 16, dans lequel lesdites peptides formant un zipper de leucine sont sélectionnées parmi le groupe composé de peptides interagissant avec WinZip A1 et des peptides interagissant avec WinZip B1.
